# EUROPÄISCHE PATENTSCHRIFT

(11) **EP 3 129 079 B1**
(45) Veröffentlichungstag und Bekanntmachung des Hinweises auf die Patenterteilung: **28.08.2019**
(21) Anmeldenummer: 15718343.5
(22) Anmeldetag: 11.03.2015
(51) Int. Cl.: A61M 1/16, A61M 1/36

(54) **OXYGENATOR MIT EINEM HOHLFASERFILMBÜNDEL**
OXYGENATOR COMPRISING A HOLLOW-FIBRE FILM BUNDLE
OXYGÉNATEUR DOTÉ D'UN FAISCEAU DE FILMS EN FIBRES CREUSES

(30) Priorität: 11.04.2014 DE 102014005353
(43) Veröffentlichungstag der Anmeldung: 15.02.2017
(73) Patentinhaber: MEDOS Medizintechnik AG, 74076 Heilbronn (DE)
(72) Erfinder: OCHEL, Wolfgang, 74252 Massenbachhausen (DE); MAURER, Andreas, 72072 Tübingen (DE)
(74) Vertreter: Castell, Klaus
(86) Internationale Anmeldenummer: PCT/DE2015/000110
(87) Internationale Veröffentlichungsnummer: WO 2015/154736

(56) Entgegenhaltungen:
- EP-A1- 2 383 001
- WO-A1-00/06357
- JP-A- 2007 215 992
- US-A1- 2007 166 190
- US-A1- 2012 277 654
- TAIJIRO SUEDA ET AL: "DEVELOPMENT OF AN OUTSIDE FLOW MEMBRANE OXYGENATOR USING A SILICONE HOLLOW FIBER", ASAIO JOURNAL, LIPPINCOTT WILLIAMS & WILKINS / ASAIO, HAGERSTOWN, MD, US, Bd. 39, Nr. 3, 1. Juli 1993 (1993-07-01), Seiten M457-M460, XP000412615, ISSN: 1058-2916

## Beschreibung

Die Erfindung betrifft einen Oxygenator mit einem Hohlfaserfilmbündel, das zumindest bereichsweise von einem Blasenrückhaltefilter umgeben ist.

Oxygenatoren werden für einen Gasaustausch zwischen einem Blutstrom und einem Gasstrom verwendet. Hierfür haben Oxygenatoren Hohlfaserfilmbündel, die eine Übergangsmembran zwischen Gasstrom und Blutstrom bilden. In der Regel wird innerhalb der Hohlfasern des Hohlfasermembranbündels das Gas geleitet, während außerhalb der Hohlfasern ein Blutstrom geleitet wird. Dadurch wird ein Gasaustausch zwischen Blutstrom und Gasstrom an der Hohlfasermembranwand erreicht. Ein derartiger Oxygenator ist beispielsweise aus der EP 765 683 bekannt, auf die vollinhaltlich Bezug genommen wird.

Sofern sich innerhalb des Blutstromes im Blut Luftblasen befinden, werden diese Luftblasen vom Eingang des Oxygenators bis zum Ausgang gefördert. Um derartige Luftblasen zurückzuhalten wird in der EP 1 839 691 B1 ein Filterelement vorgeschlagen, das eine Umfangselastizität besitzt und unter Spannung am Hohlfasermembranbündel anliegt. Auch die EP 1 618 906 B1 beschreibt einen derartigen Oxygenator, bei dem eine Innenumfangsfläche eines ringförmigen Blasenabfangfilterelementes in Kontakt mit einer Außenumfangsfläche des röhrenförmigen Hohlfaserfilmbündels steht.

Es hat sich herausgestellt, dass die Verwendung derartiger Blasenrückhaltefilter die Funktion des Oxygenators negativ beeinflusst.

In Taijiro Sueda et al.: Development of an outside Flow Membrane Oxygenator using a Silicone Hollow Fiber" Asaio Journal, Lippincott Williams & Wilkins /Asaio, Hagerstown, US, Bd. 39, Nr. 3, 1. Juli 1993 (1993-07-01), Seiten M457-M460, XP000412615, ISSN: 1058-2916 wird ein gattungsgemäßer Oxygenator beschrieben, bei dem das Hohlfaserfilmbündel von einem Polyurethan Schaum umgeben ist und ein Polyesterfilter als Blasenrückhaltefilter verwendet wird. Einen ähnlichen Oxygenator zeigt die US 2007/166190 A1.

Der Erfindung liegt daher die Aufgabe zu Grunde, einen gattungsgemäßen Oxygenator so weiter zu entwickeln, dass das Blasenrückhaltefilter die Funktion des Oxygenators nicht beeinträchtigt.

Diese Aufgabe wird mit einem gattungsgemäßen Oxygenator gelöst, bei dem die Haltestruktur flexibel ist und auf das Hohlfaserfilmbündel einen Kompressionsdruck ausübt, sodass zwischen der Haltestruktur und dem Blasenrückhaltefilter ein Zwischenraum vorhanden ist.

Um zwischen Blasenrückhaltefilter und gasdurchlässiger Haltestruktur einen Zwischenraum zu schaffen, ist die Haltestruktur flexibel und übt auf das Hohlfaserfilmbündel einen Kompressionsdruck aus. Da die Haltestruktur keinen definierten Porendurchmesser aufweisen muss, kann sie flexibel gestaltet werden und das Hohlfaserfilmbündel komprimieren. Das Blasenrückhaltefilter sollte hingegen nicht gedehnt werden, da sich dadurch der Porendurchmesser vergrößert, wodurch die Funktion des Blasenrückhaltefilters beeinträchtigt werden kann.

Die gasdurchlässige Haltestruktur verhindert einen Kontakt zwischen Blasenrückhaltefilter und Hohlfaserfilmbündel. Die Hohlfasern des Hohlfaserfilmbündels können somit nicht mehr direkt auf den Filteröffnungen des Blasenrückhaltefilters liegen und dadurch wird vermieden, dass die Funktion des Blasenrückhaltefilters durch an ihn anliegende Hohlfasern beeinträchtigt wird. Auch die Funktion der Hohlfasern an der Außenseite des Hohlfaserfilmbündels wird in Folge der gasdurchlässigen Haltestruktur nicht mehr durch ein an der Hohlfaser anliegendes Blasenrückhaltefilter beeinträchtigt. Die Haltestruktur führt daher zu dem Ergebnis, dass die volle Funktionsfähigkeit des Blasenrückhaltefilters ausgenutzt werden kann und auch die radial außenliegenden Hohlfasern vom Blut umströmt werden können.

Die gasdurchlässige Haltestruktur lässt auch das Blut passieren, sodass das Blut, das zunächst am Hohlfaserfilmbündel für einen Gasaustausch entlang geführt wurde, anschließend zuerst durch die gasdurchlässige Haltestruktur und anschließend durch das Blasenrückhaltefilter fließt. Die Blasen können somit ohne Beeinträchtigung der Funktion des Oxygenators zurückgehalten werden.

Vorteilhaft ist es, wenn die gasdurchlässige Haltestruktur am Hohlfasermembranbündel anliegt. Da durch die gasdurchlässige Haltestruktur ein Anliegen des Blasenrückhaltefilters am Hohlfasermembranbündel verhindert ist, kann die gasdurchlässige Haltestruktur am Hohlfaserfilmbündel anliegen. Hierfür kann die gasdurchlässige Haltestruktur derart ausgebildet sein, dass sie die Funktion des Hohlfaserfilmbündels nicht beeinträchtigt.

Um flächig mit dem Hohlfaserfilmbündel zusammen zu wirken, wird vorgeschlagen, dass die Haltestruktur die Außenfläche des Hohlfaserfilmbündels umgibt. Diese Außenfläche ist gebogen oder planflächig, je nach Ausbildung des Oxygenators. Bei gestapelten Membranen ist die Außenfläche in der Regel planflächig und bei gewickelten Oxygenatoren ist die Außenfläche in der Regel gebogen oder radial zu einer zentralen Linie angeordnet.

Eine Ausführungsvariante sieht vor, dass die Haltestruktur ein Netz ist. Ein derartiges Netz kann mit einem definierten Porendurchmesser hergestellt werden und es gewährleistet eine große freie Durchgangsfläche in Relation zur benötigten Materialmenge.

Versuche haben gezeigt, dass es vorteilhaft ist, wenn die Haltestruktur eine Porenweite von etwa 100 Mikrometer und vorzugsweise mehr als 100 Mikrometer aufweist. Das Blasenabfangfilter sollte hingegen eine Porenweite von nicht mehr als 50 Mikrometer aufweisen, um sicher zu funktionieren.

Vorteilhaft ist es dabei, wenn das Blasenrückhaltefilter und die gasdurchlässige Struktur jeweils aus einem Gewebe hergestellt sind. Blasenrückhaltefilter und gasdurchlässige Struktur sind vorzugsweise zwei getrennt voneinander hergestellte Einzelteile, die die jeweils als Gewebe aus den gleichen oder unterschiedlichen Materialien hergestellt werden können.

Bei einem Ausführungsbeispiel werden das Blasenrückhaltefilter und die gasdurchlässige Struktur aus demselben Material und vorzugsweise aus einem Polymer, vorzugsweise aus Polyester (PET) hergestellt. Bei einer alternativen Ausführungsvariante wird die gasdurchlässige Haltestruktur aus einem Polymer, vorzugsweise aus Polyamid (PA) hergestellt.

Eine vorteilhafte Ausführungsvariante sieht vor, dass die gasdurchlässige Struktur eine konische Form aufweist, die der Form einer radial außerhalb des Hohlfaserfilmbündels angeordneten Innenwandung des Oxygenators entspricht. Die konische Form der gasdurchlässigen Struktur erlaubt es, die gasdurchlässige Struktur in eine konische Innenwandung des Oxygenators soweit einzuführen, bis sie flächig an der Innenwandung des Oxygenators anliegt. Hierzu ist es vorteilhaft, wenn die gasdurchlässige Struktur eine gewisse Eigenstabilität aufweist, die es erlaubt, die gasdurchlässige Struktur so in den von der konischen Innenwandung des Oxygenators gebildeten Raum einzuführen, dass sie sich beim Einfuhren an die Innenwandung des Oxygenators anlegt.

Ein Ausführungsbeispiel eines Oxygenators ist in der Zeichnung dargestellt und wird im Folgenden beschrieben.

Es zeigt
- Figur 1: eine perspektivische Ansicht eines Oxygenators,
- Figur 2: eine Draufsicht auf den Oxygenator,
- Figur 3: einen Schnitt durch den in Figur 1 gezeigten Oxygenator und
- Figur 4: schematisch das Zusammenwirken von Blasenrückhaltefilter und gasdurchlässiger Haltestruktur.

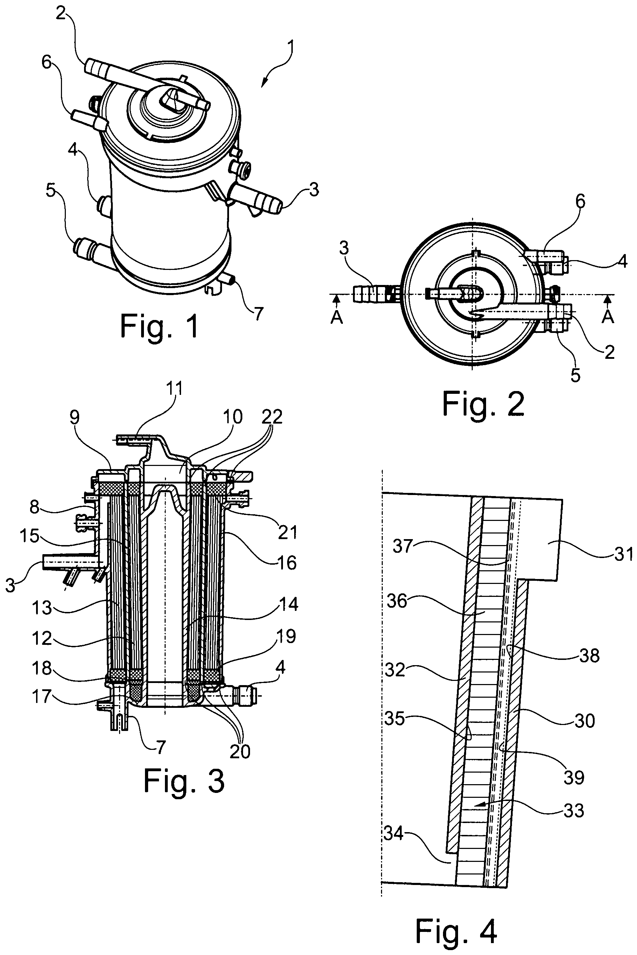

Der in Figur 1 gezeigte Oxygenator 1 hat einen Bluteinlass 2 und einen Blutauslass 3. Als weitere Anschlüsse sind ein Wassereinlass 4 und ein Wasserauslass 5 zum Temperieren des Blutes vorgesehen. Gas wird dem Oxygenator am Einlass 6 zugeführt und am Auslass 7 abgeführt

Das Oxygenatorgehäuse 8 hat einen Deckel 9, über den das Blut zugeführt wird. Sofern sich im zugeführten Blut Luftblasen befinden sollten, werden diese durch einen erzwungenen Blutstrudel im Eingangsbereich 10 von der Flüssigkeit getrennt und durch den Gasauslass 11 abgeführt.

Das Blut strömt zunächst in radial inneren Bereich entlang einem Hohlfaserfilmbündel 12, in dem Wasser zum Temperieren des Blutes geführt ist und anschließend durch einen radial äußeren Bereich, in dem ein Hohlfasermembranbündel 13 angeordnet ist, um durch in den Hohlfasern geführtes Gas einen Gasaustausch zwischen Blut und Gasströmung zu erzielen. Letztlich verlässt das Blut den Oxygenator durch den Ausgang 3.

Das Oxygenatorgehäuse hat eine radial innenliegende Wandung 14, eine mittlere Wandung 15 und eine äußere Wandung 16. Der untere Bereich des Oxygenators wird von einem Boden 17 gebildet, in den Wasserzulauf und -ablauf 4, 5 und der Gasablauf 7 angeordnet sind.

Die Hohlfasermembranbündel 12 und 13 sind zur Abdichtung an den Stellen 18 bis 22 in Kleber eingebettet.

Die Figur 4 zeigt sehr schematisch und nur beispielhaft, eine äußere Gehäusewandung 30 mit einem Blutauslassbereich 31 und eine radial weiter innen angeordnete innere Gehäusewandung 32. Äußere und innere Gehäusewandung sind leicht konisch und konzentrisch zueinander angeordnet, sodass zwischen den Gehäusewandungen ein Raum 33 liegt, der von parallel zueinander angeordneten Wandung radial begrenzt ist. Im unteren Bereich der inneren Wandung 32 befindet sich ein Einlass 34, der es ermöglicht, dass Blut vom Einlass 34 durch den Raum 33 zum Auslass 31 fließt.

Im Raum 33 ist an der Außenseite 35 der inneren Wandung 32 anliegend ein Hohlfasermembranbündel 36 vorgesehen. Dieses Hohlfasermembranbündel 36 wird durch eine gasdurchlässige Haltestruktur 37 nach radial innen an die innere Wandung 32 gedrückt. Die gasdurchlässige Haltestruktur wirkt daher wie eine Hohlfaserkompressionsschicht.

An der anderen Seite des Raumes 33 liegt an der Innenseite 38 der äußeren Wandung 30 ein Blasenrückhaltefilter 39 an, der an einer Stelle durchströmt werden muss, damit das Blut vom Bluteinlass 34 zum Blutauslass 31 gelangen kann.

Bei einem Oxygenator 1 wie er in Figur 1 dargestellt ist, werden die gegenüberliegenden Enden des Blasenrückhaltefilters 39 und der gasdurchlässigen Haltestruktur 37 an den Stellen 18 bis 22 wie die Hohlfasermembranbündel 12 und 13 in Kleber eingebettet und anschließend an Kopf- und Bodenseite zusammen mit dem überstehenden Kleber abgeschnitten.

Im eingebauten Zustand steht die gasdurchlässige Haltestruktur 37 derart unter Spannung, dass sie das Hohlfaserfilmbündel 36 gegen die innere Wandung 32 presst. Dabei werden die Öffnungen in der netzartigen Haltestruktur durch die Spannung offen gehalten, sodass eine Porenweite mit einem mittleren Durchmesser der Poren von mindestens 100 Mikrometer entsteht.

Beabstandet zur Haltestruktur 37 liegt das Blasenrückhaltefilter 39, dessen Porenweite so ausgelegt ist, dass der durchschnittliche Durchmesser der Poren nicht mehr als 50 Mikrometer beträgt.

## Patentansprüche

1. Oxygenator (1) mit einem Hohlfaserfilmbündel (13, 36), das zumindest bereichsweise von einem Blasenrückhaltefilter (39) umgeben ist, wobei zwischen Hohlfaserfilmbündel (13, 36) und Blasenrückhaltefilter eine gasdurchlässige Haltestruktur (37) angeordnet ist, wobei die Haltestruktur (37) flexibel ist und auf das Hohlfaserfilmbündel (13, 36) einen Kompressionsdruck ausübt, sodass zwischen der Haltestruktur und dem Blasenrückhaltefilter ein Zwischenraum vorhanden ist.

2. Oxygenator nach Anspruch 1, wobei die gasdurchlässige Haltestruktur (37) am Hohlfaserfilmbündel (13, 36) anliegt.

3. Oxygenator nach Anspruch 1 oder 2, wobei die Haltestruktur (37) die Außenfläche des Hohlfaserfilmbündels (13, 36) umgibt.

4. Oxygenator nach einem der vorhergehenden Ansprüche, wobei die Haltestruktur (37) ein Netz ist.

5. Oxygenator nach einem der vorhergehenden Ansprüche, wobei die Haltestruktur (37) eine Porenweite von etwa 100 Mikrometer und vorzugsweise mehr als 100 Mikrometer aufweist.

6. Oxygenator nach einem der vorhergehenden Ansprüche, wobei das Blasenrückhaltefilter (39) eine Porenweite von nicht mehr als 50 Mikrometer aufweist.

7. Oxygenator nach einem der vorhergehenden Ansprüche, wobei Blasenrückhaltefilter (39) und gasdurchlässige Haltestruktur (37) aus jeweils einem Gewebe hergestellt sind.

8. Oxygenator nach einem der vorhergehenden Ansprüche, wobei Blasenrückhaltefilter (39) und gasdurchlässige Haltestruktur (37) aus demselben Material und vorzugsweise aus einem Polymer, vorzugsweise aus Polyester hergestellt sind.

9. Oxygenator nach einem der vorhergehenden Ansprüche, wobei die gasdurchlässige Haltestruktur (37) aus einem Polymer, vorzugsweise aus Polyamid hergestellt ist.

10. Oxygenator nach einem der vorhergehenden Ansprüche, wobei die gasdurchlässige Haltestruktur (37) eine konische Form aufweist, die der Form einer radial außerhalb des Holfaserfilmbündels (13, 36) angeordneten Innenwandung (39) des Oxygenators entspricht.

## Claims

1. An oxygenator (1) with a hollow-fibre film bundle (13, 36) which at least in sections is surrounded by a bubble retention filter (39), wherein between hollow-fibre film bundle (13, 36) and bubble retention filter (39) a gas-permeable supporting structure (37) is arranged, wherein the supporting structure (37) is flexible and exerts a compression pressure on the hollow-fibre film bundle (13, 36) so that an intermediate space is present between the supporting structure and the bubble retention filter.

2. The oxygenator according to claim 1, wherein the gas-permeable supporting structure (37) is in contact on the hollow-fibre film bundle (13, 36).

3. The oxygenator according to claim 1 or 2, wherein the supporting structure (37) surrounds the outer surface of the hollow-fibre bundle (13, 36).

4. The oxygenator according to any one of the preceding claims, wherein the supporting structure (37) is a net.

5. The oxygenator according to any one of the preceding claims, wherein the supporting structure (37) has a pore width of around 100 micrometres and preferably more than 100 micrometres.

6. The oxygenator according to any one of the preceding claims, wherein the bubble retention filter (39) has a pore width of no more than 50 micrometres.

7. The oxygenator according to any one of the preceding claims, wherein the bubble retention filter (39) and the gas-permeable supporting structure (37) are each made of a woven fabric.

8. The oxygenator according to any one of the preceding claims, wherein bubble retention filter (39) and gas-permeable supporting structure (37) are made of the same material and preferably of a polymer, preferably polyester.

9. The oxygenator according to any one of the preceding claims, wherein the gas-permeable supporting structure (37) is made of a polymer, preferably of polyamide.

10. The oxygenator according to any one of the preceding claims, wherein the gas-permeable structure (37) is of a conical shape which corresponds to the shape of an inner wall (39) of the oxygenator arranged radially outside the hollow-fibre film bundle (13, 36).

## Revendications

1. Oxygénateur (1) doté d'un faisceau de films en fibres creuses (13, 36) qui est entouré au moins par endroits par un filtre de retenue de bulles (39), une structure de retenue perméable au gaz (37) étant disposée entre le faisceau de films en fibres creuses (13, 36) et le filtre de retenue de bulles, la structure de retenue (37) étant souple et exerçant une force de compression sur le faisceau de films en fibres creuses (13, 36), de sorte qu'un intervalle est présent entre la structure de retenue et le filtre de retenue de bulles.

2. Oxygénateur selon la revendication 1, dans lequel la structure de retenue perméable au gaz (37) repose au niveau du faisceau de films en fibres creuses (13, 36).

3. Oxygénateur selon la revendication 1 ou 2, dans lequel la structure de retenue (37) entoure la surface extérieure du faisceau de films en fibres creuses (13, 36).

4. Oxygénateur selon une des revendications précédentes, dans lequel la structure de retenue (37) est un filet.

5. Oxygénateur selon une des revendications précédentes, dans lequel la structure de retenue (37) présente une largeur de pores d'environ 100 micromètres et de préférence de plus de 100 micromètres.

6. Oxygénateur selon une des revendications précédentes, dans lequel le filtre de retenue de bulles (39) présente une largeur de pores qui n'excède pas 50 micromètres.

7. Oxygénateur selon une des revendications précédentes, dans lequel le filtre de retenue de bulles (39) et la structure de retenue perméable au gaz (37) sont fabriqués respectivement à partir d'un textile.

8. Oxygénateur selon une des revendications précédentes, dans lequel le filtre de retenue de bulles (39) et la structure de retenue perméable au gaz (37) sont fabriqués à partir du même matériau et de préférence à partir d'un polymère, de préférence du polyester.

9. Oxygénateur selon une des revendications précédentes, dans lequel la structure de retenue perméable au gaz (37) est fabriquée à partir d'un polymère, de préférence du polyamide.

10. Oxygénateur selon une des revendications précédentes, dans lequel la structure de retenue perméable au gaz (37) présente une forme conique qui correspond à la forme d'une paroi intérieure (39) disposée radialement à l'extérieur du faisceau de films en fibres creuses (13, 36) de l'oxygénateur.
